# EUROPEAN PATENT SPECIFICATION

(11) **EP 0 514 917 B1**
(45) Date of publication and mention of the grant of the patent: **27.12.1996**
(21) Application number: 92108656.7
(22) Date of filing: 22.05.1992
(51) Int. Cl.: C07D 487/04, C07D 239/84

(54) **Process for and 2-(cyanoimino)-quinazoline derivatives useful as intermediates in the preparation of 6,7-di-(chloro)-1,5-di(hydro)-imidazo-[2,1-b]quinazolin-2[3H]-one and process for preparing the 2-(cyanoimino)-quinazoline derivatives**
Prozess und 2-(Cyanoimino)-Quinazlin-Zwischenprodukte zur Herstellung von 6,7-Di(Chloro)-1,5-Di(Hydro)-Imidazo-[2,1-b]Quinazolin-2[3H]-on sowie Prozess zur Darstellung der 2-(Cyanoimino)Quinazolin
Procédé et intermediaires de 2-(cyanoimino)-quinazoline pour la préparation de 6,7-di-(chloro)-1,5-di(hydro)-imidazo-[2,1-b]quin-azolin-2[3]-one aissi que procédé de préparation de 2-(cyanoimino)-quinazoline

(30) Priority: 22.05.1991 HU 170791; 22.05.1991 HU 170891
(43) Date of publication of application: 25.11.1992
(73) Proprietor: EGIS GYOGYSZERGYAR, Budapest X (HU)
(72) Inventor: Reiter, Józef, Dr., H-1022 Budapest (HU); Trinka, Péter, H-1116 Budapest (HU); Tömpe, Péter, Dr., H-1116 Budapest (HU); Szabó, Eva, Dr., H-1162 Budapest (HU); Slégel, Péter, Dr., H-1067 Budapest (HU); Brlik, János, H-1154 Budapest (HU); Halbauer, Agnes, H-1046 Budapest (HU); Sztruhár, Ilona, Dr., H-1191 Budapest (HU); Kenyeres, Magdolna, H-1165 Budapest (HU); Görgényi, Frigyes, Dr., H-1115 Budapest (HU); Csörgö, Margit, Dr., H-1174 Budapest (HU); Zsarnúczai, Szvetlana, H-1172 Budapest (HU); Benkö, Sarolta, H-1024 Budapest (HU); Gigler, Gábor, H-1061 Budapest (HU); Danyi, Dezsö, H-1141 Budapest (HU); Fekete, Pál, Dr., H-1051 Budapest (HU); Király, Mária, H-1161 Budapest (HU)
(74) Representative: Beszédes, Stephan G., Dr.

(56) References cited:
- EP-A- 0 406 958
- US-A- 4 208 521
- CHEMICAL AND PHARMACEUTICAL BULLETIN. vol. 28, no. 5, 1980, TOKYO JP pages 1357 - 1364; F. ISHIKAWA: 'Cyclic Guanidines. IX. Synthesis of 2-Amino-3,4- dihydroquinazolines as Blood Platelet Aggregation Inhibitors'

## Description

This invention is concerned with a new and improved process for the preparation of 6,7-di-(chloro)-1,5-di-(hydro)-imidazo[2,1-b]-quinazolin-2[3H]-one of the formula 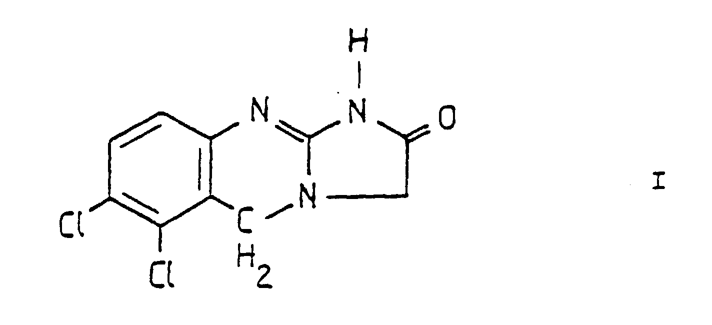 [anagrelide], with 2-(cyanoimino)-quinazoline derivatives as new intermediates useful in the preparation of this compound and with a process for the preparation of the said new intermediates.

It is known that 6,7-di-(chloro)-1,5-di-(hydro)-imidazo-[2,1-b]-quinazolin-2[3H]-one [anagrelide] is a valuable blood platelet antiaggregative compound which has been recently reported to be a potent inhibitor of cyclic adenosine monophosphate phosphodiesterase.

Several methods are known for the preparation of 6,7-di--(chloro)-1,5-di-(hydro)-imidazo[2,1-b]-quinazolin-2[3H]-one [anagrelide].

The U.S. patent specification No. 3,932,407 discloses a process for the preparation of 6,7-di-(chloro)-1,5-di-(hydro)--imidazo[2,1-b]-quinazolin-[3H]-one hydrobromide [anagrelide hydrobromide] which comprises boiling a diamine of the general formula 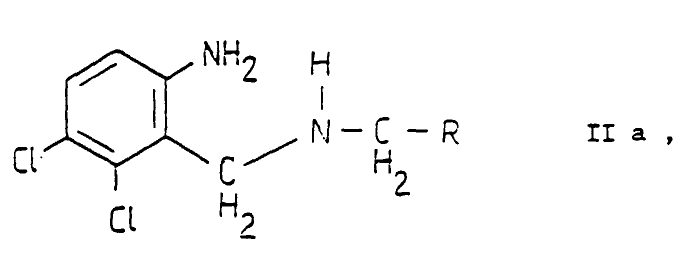 wherein
R is an ester group,
with cyanogen bromide in ethanol. The reaction time is rather long, it lasts for 22 hours. An improved method is disclosed in the U.S. patent specification No. 4,146,718. According to this method the diamine of the general formula IIa is boiled with cyanogen chloride, cyanogen bromide or cyanogen iodide for a long time, i.e. for 12 to 18 hours, to yield as intermediate a 2-iminoquinazoline salt of the general formula 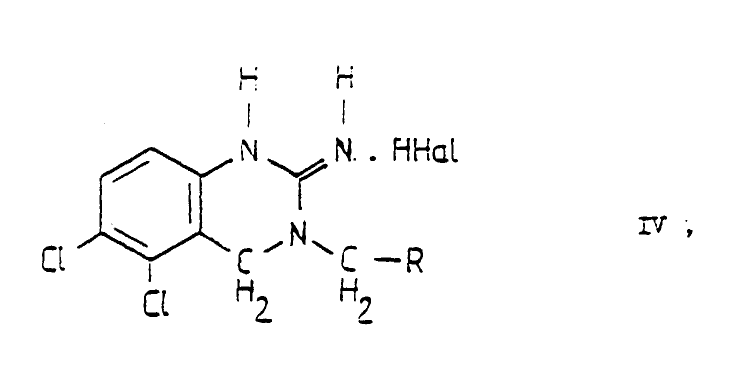 wherein
R stands for an ester group and
HAL represents halogen,
and the base liberated from this intermediate is then boiled for 4 hours in ethanol to obtain 6,7-di-(chloro)-1,5-di-(hydro)-imidazo[2,1-b]-quinazolin-2[3H]-one [anagrelide].

A serious drawback of both methods resides in the application of extremely toxic cyanogen halides, requiring special technology and special equipments on an industrial scale production.

According to the process described in the U.S. patent specification No. 3,932,407 a diamine of the general formula II is subjected to cyclization with 1,1-carbonyldiimidazole, the corresponding quinazolin-2-one ester of the general formula 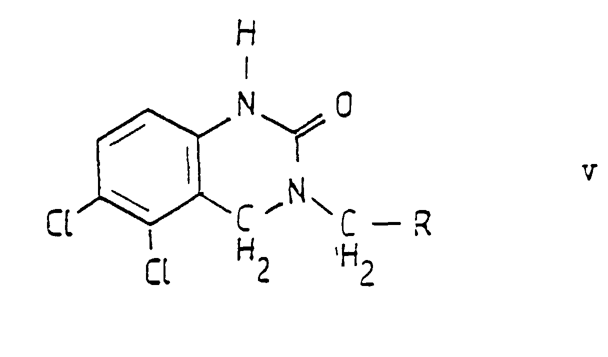 wherein
R is an ester group,
thus obtained is reacted with phosphorus oxychloride to yield the corresponding quinazoline ester of the general formula 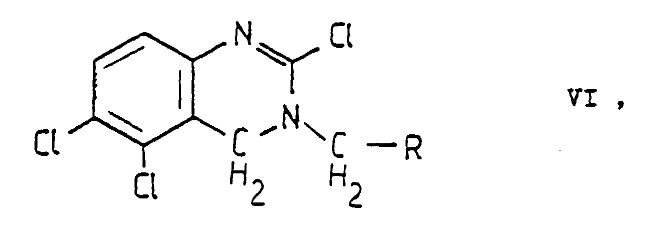 wherein
R is an ester group,
and this latter compound is boiled with ethanolic ammonia to obtain 6,7-di-(chloro)-1,5-di-(hydro)-imidazo[2,1-b]--quinazolin-2[3H]-one [anagrelide]. The disadvantages of this process reside in the facts that 1,1-carbonyldiimidazole is a difficultly available substance, and when reacting a quinazolin-2-one ester of the general formula V with phosphorus oxychloride, the acid-sensitive ester group decomposes, causing the formation of a considerable amount of by-products. This is the reason why the patent specification discloses only the yield of the crude product and is silent about the losses of purification. The closing step of this synthesis is also disadvantageous since the reaction with ethanolic ammonia is to be carried out at an elevated pressure and the reaction time is rather long, i.e. 16 hours.

The cyclization of a compound of the general formula VI,
in which
R is ethoxycarbonyl,
with ethanolic ammonia, is described by Japanese authors in J. Heterocyclic chem., 18, 67 (1981), too, but this reaction is also carried out at an elevated pressure in a closed bomb tube, at a temperature of 120°C for 16 hours, and hence this process is also disadvantageous for an industrial scale production.

The U.S. patent specification No. 3,932,407 describes a further process for the preparation of the 6,7-di-(chloro)-1,5-di-(hydro)-imidazo[2,1-b]-quinazolin-2[3H]-one [anagrelide] of formula I. According to this process a diamine of the general formula 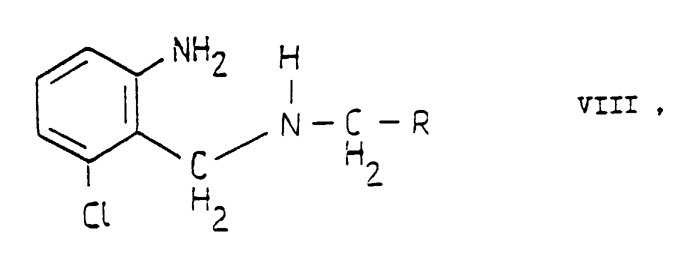 wherein
R is an ester group,
is reacted with a cyanogen halide to form a quinazolinone of the general formula 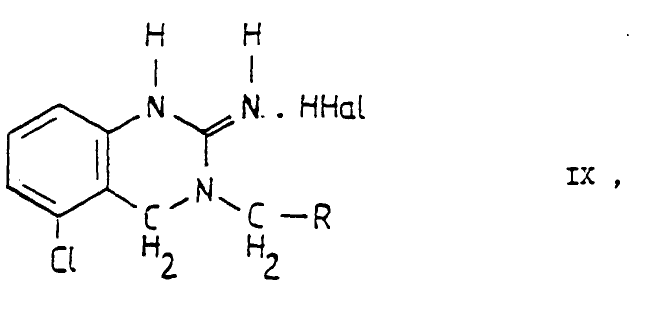 wherein
R is an ester group,
which is then cyclized into an imidazoquinazolinone derivative of the general formula 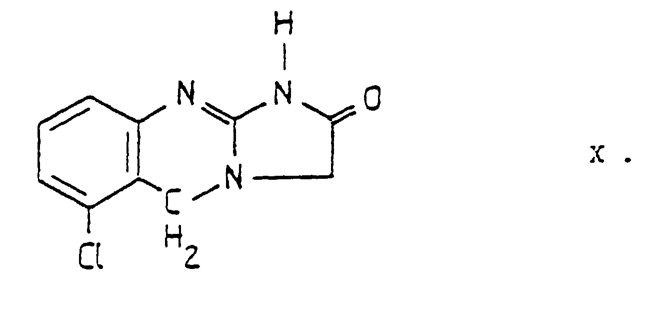 The latter compound is chlorinated at an elevated pressure, in the presence of anhydrous iron chloride to obtain 6,7-di-(chloro)-1,5-di-(hydro)-imidazo[2,1-b]-quinazolin-2[3H]-one [anagrelide]. A serious drawback of this method, in addition to the foregoing ones, resides in the fact that during the chlorination process not only the desired 6,7-di-(chloro)-derivative is obtained, but further dichloro isomers, chlorinated in other positions of the aromatic ring, are also formed, and the separation thereof from the 6,7-di-(chloro)-1,5-di-(hydro)-imidazo[2,1-b]-quinazolin-2[3H]-one [anagrelide] of formula I is rather cumbersome. This may be the reason why the patent specification is silent about the isolation, identification and even the melting point of the 6,7-di-(chloro)-1,5-di-(hydro)-imidazo[2,1-b]-quinazolin-2[3H]-one [anagrelide] of the formula I, only the 6-(chloro)-7-(bromo) analogue thereof is described, but yield data are given only for the crude product and neither the losses of purification nor the separation of the isomers are mentioned.

According to the method described in the U.S. patent specification No. 4,208,521 a diamine of the general formula IIa,
wherein
R stands for an ester group,
is reacted with a guanile derivative of the general formula 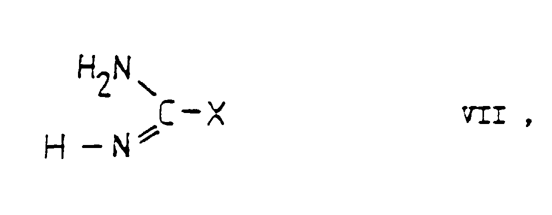 wherein
X represents a leaving group,
to obtain directly 6,7-di-(chloro)-1,5-di-(hydro)-imidazo-[2,1-b]-quinazolin-2[3H]-one [anagrelide]. As leaving group amino, alkylthio, arylthio and optionally substituted nitrogen-containing heterocyclic groups are mentioned, but as regards the preparation of 6,7-di-(chloro)-1,5-di-(hydro)-imidazo-[2,1-b]-quinazolin-2[3H]-one only the more reactive 1-(guanile)-3,5-di-(methyl)-pyrazole nitrate is specified, which is boiled for as long as 24 hours to obtain 6,7-di-(chloro)-1,5-di-(hydro)-imidazo[2,1-b]-quinazolin-2[3H]-one [anagrelide] with a yield of 40%. The very long reaction time and the low yield and particularly the low purity of the desired product because of formation of a side-product difficultly separable from the desired product render this process uneconomical for industrial scale production. In case of other carboxamidine derivative reactants the reaction times and yields are still more unfavorable even with starting substances not containing chlorine atoms in the benzene ring.

From EP-A-O 406 958, particularly page 4, line 36 to page 5, line 26 it has been known to prepare 1,5-di-(hydro)-imidazo[2,1-b]-quinazolin-2[3H]-ones substituted in the 6-position by an acyl group, an oxyme group or a carboxy alkenyl group, optionally substituted, by thermic cyclization of [2-(cyanoimino-1,3,4-tetra-(hydro)-quinazolin-3-yl]-carbonic acid alkylesters in the presence of acids. As solvents alkanols, e.g. ethanol, propanol or butanol, have been indicated. No example has been described for this reaction.

Further in EP-A-O 406 958 it has been described the reaction of [2-(amino)-benzylamino]-carbonic acid alkylesters, substituted in the 6-position by an acyl group, an oxyme group or a carboxyalkenyl group, optionally substituted, with N-cyanoimido-S,S-dimethyldithiocarbonate or with an O-alkylisourea or S-alkylisothiourea to form [2-(cyanoimino)-1,2,3,4-tetra-(hydro)-quinazolin-3-yl]-carbonic acid alkylesters, substituted in the 6-position by an acyl group, an oxyme group or a carboxyalkenyl group, optionally substituted. No particulars of this reaction have been indicated.

Particulars of such reaction can be taken from Chemical and Pharmaceutical Bulletin, Vol. 28, No. 5, 1980, pages 1357 to 1364, particularly page 1363, second paragraph. There it has been described the reaction of N-[2-(amino)-benzylamino]-N-[benzyl]-amine with dimethyl cyanoimidodithiocarbonate at 180 to 200°C (in the melt) for 30 minutes to form 3-benzyl-2-cyanoimino-1,2,3,4-tetrahydroquinazoline in a yield of 57%.

The problem underlying to the present invention is to create a simple process which enables the favourable preparation of the 6,7-di-(chloro)-1,5-di-(hydro)-imidazo[2,1-b]-quinazolin-2[3H]-one [anagrelide] of the formula I.

A subject-matter of the present invention is a process for the preparation of 6,7-di-(chloro)-1,5-di-(hydro)-imidazo[2,1-b]quinazolin-2[3H]-one of formula 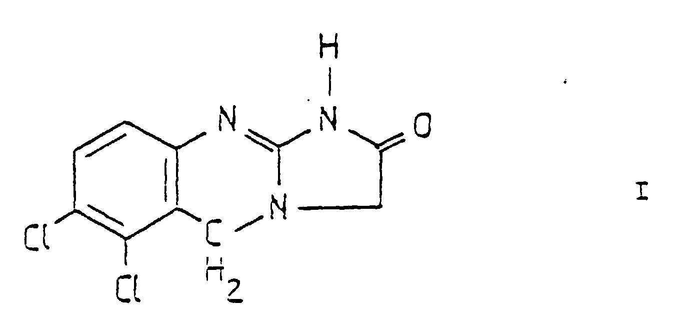 or a salt thereof including their hydrates, which is charactarized by subjecting [2-(cyanoimino)-5-,6-di-(chloro)-1,2,3,4-tetra-(hydro)-quinazolin-3-yl]-acetonitrile or a [2-(cyanoimino)-5,6-di-(chloro)-1,2,3,4-tetra-(hydro)-quinazolin-3-yl]-acetic acid alkylester of the general formula 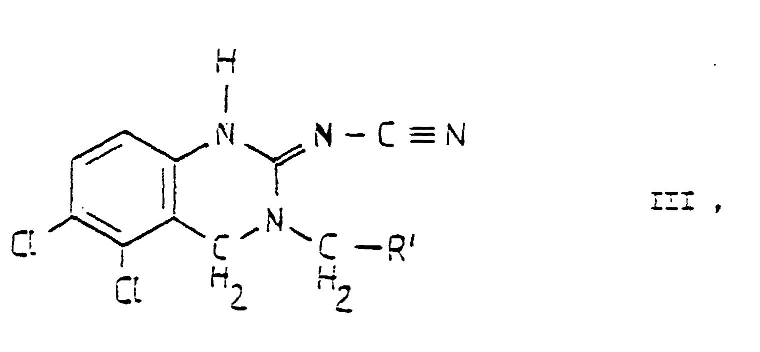 wherein
R' stands for a cyano group or a group of the fomula 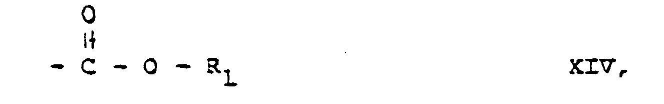 in which latter
R₁ represents a straight or branched alkyl group having from 1 to 6 carbon atom(s), optionally carrying a phenyl subsequent,
to thermic cyclization in an acidic medium with ethylene glycol, ethylene glycol dimethyl ether, ethylene glycol diethyl ether, diethylene glycol dimethyl ether, diethylene glycol diethyl ether, 2-(methoxy)-ethanol, 2-(ethoxy)-ethanol, N-(methyl)-pyrrolidone, dimethylformamide, acetonitrile and/ /or dioxane and/or in an aliphatic organic acid having from 1 to 4 carbon atom(s) as [a] solvent(s).

The hydrates of the 6,7-di-(chloro)-1,5-di-(hydro)-imidazo[2,1-b]-quinazolin-2[3H]-one [anagrelide] may be e.g. semihydrates and monohydrates.

Examples for the alkyl group represented by R₁ are methyl, ethyl, propyl, isopropyl, butyl, tert.-butyl, pentyl and hexyl groups.

Preferably the alkyl group represented by R₁ is such having from 1 to 4 carbon atom(s). These groups may be substituted by one phenyl group at any carbon atom.

According to an advantageous embodiment of the invention the cyclization is carried out in a mixture of 1 or more solvent(s) inert towards the reactants and a mineral acid.

Preferably it is boiled for 20 to 180 minutes.

The above solvents are such having a boiling point exceeding 80°C.

As a mineral acid preferably hydrogen chloride is used.

Particularly as an aliphatic organic acid having from 1 to 4 carbon atom(s) acetic acid is used.

In cases in which the cyclization is performed by using as a solvent an aliphatic organic acid there is no need for using a mineral acid but additionally also the latter can be used.

The thermic cyclization is preferably carried out at a temperature from 80°C to 130°C.

When the reaction is accomplished, the reaction mixture can be cooled and the product filtered off either in form of a salt or, after neutralization, in form of the free base.

The starting [2-(cyanoimino)-5,6-di-(chloro)-1,2,3,4-tetra-(hydro)-quinazolin-3-yl]-acetonitriles and -acetic acid alkylesters of the general formula III are new compounds

Hence as a further subject matter of the present invention there are provided for new 2-(cyanoimino)-quinazoline derivatives of the general formula 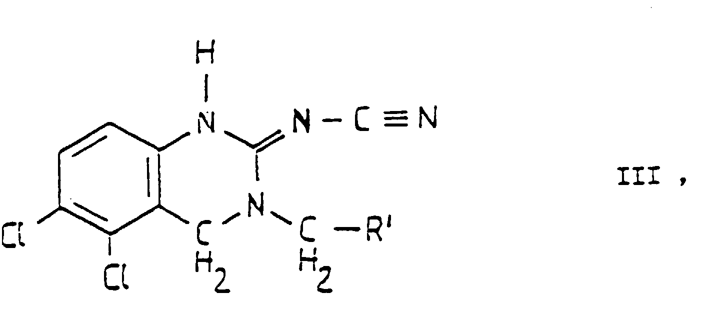 wherein
R' stands for a cyano group or a group of the formula 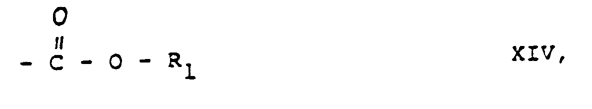 in which latter
R₁ represents a straight or branched alkyl group having from 1 to 6 carbon atom(s), optionally carrying a phenyl substituent.

Surprisingly further reacting them by the process according to the invention having peculiarity yields 6,7-di-(chloro)-1,5-di-(hydro)-imidazo[2,1-b]-quinazolin-2[3H]-one [anagrelide] with superior results.

A still further subject matter of the invention is a process for the preparation of the [2-(cyanoimino)-5,6-di-(chloro)-1,2,3,4-tetra-(hydro)-quinazolin-3-yl]-acetonitriles or -acetic acid alkylesters according to the invention which is characterized by reacting a [2-(amino)-5,6-di-(chloro)-benzylamino]-acetonitrile or a [2-(amino)-5,6-di-(chloro)-benzylamino]-acetic acid alkylester of the general formula 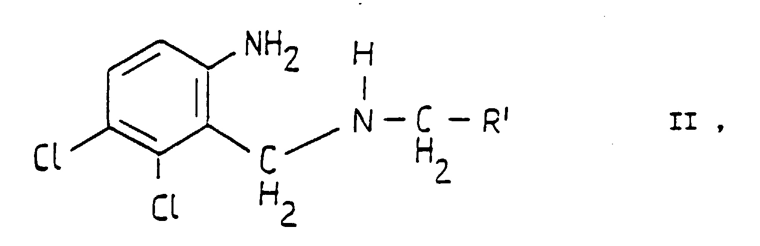 wherein
R' is as defined above,
with a cyanoimino derivative of the general formula 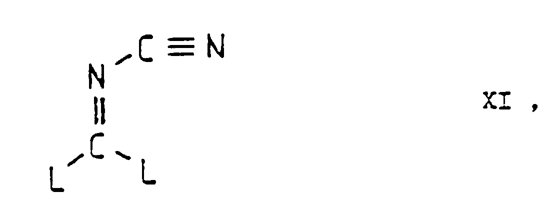 wherein
L is a leaving group.

Preferably as cyanoimino derivative of the general formula XI such one is used
in which
L stands for a group of formula

X - R₂ XV,

in which latter
- X: represents oxygen or sulfur and
- R₂: means an alkyl group having from 1 to 6, particularly 1 to 4, carbon atom(s), optionally carrying a phenyl substituent.

Suitably the reaction of the compounds of the general formulae II and XI is carried out in [a] solvent(s) inert towards the reactants, particularly in an alcohol having from 1 to 8 carbon atom(s), most particularly in such having from 1 to 4 carbon atom(s), and/or [an] apolar and/or polar aprotic solvent(s). Above all isopropanol, benzene, acetonitrile and/or dimethylformamide are used as solvent(s).

Suitably the reaction is carried out at a temperature from 0°C to 100°C, preferably from 20°C to 80°C, particularly preferably at room temperature.

The [2-(cyanoimino)-5,6-di-(chloro)-1,2,3,4-(hydro)-quinazolin-3-yl]-acetonitriles or -acetic acid alkylesters of the general formula III can be isolated from the reaction mixture by methods known per se, generally by conventional filtration followed by recrystallization from an appropriate solvent.

The [2-(amino)-5,6-di-(chloro)-benzylamino]-acetonitrile and [2-(amino)-5,6-di-(chloro)-benzylamino]-acetic acid alkylesters of the general formula II used as starting substances can be prepared e.g. by subjecting the nitro and cyano groups of 2,3-di-(chloro)-6-(nitro)-benzonitrile to reduction and reacting the compound 2,3-di-(chloro)-6-(amino)-methylamine of formula 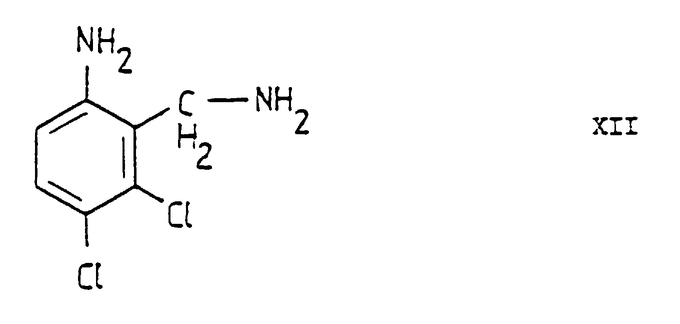 thus obtained with a haloacetic acid ester or haloacetic nitrile of the general formula 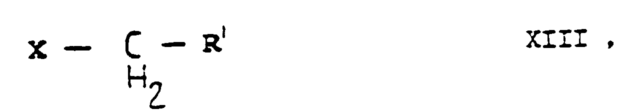 wherein
X stands for halogen and R' is as stated above.

The invention provides a process for the preparation of 6,7-di-(chloro)-1,5-di-(hydro)-imidazo[2,1-b]-quinazolin-2[3H]-one [anagrelide] of formula I which is more advantageous than the hitherto known processes due to the elimination of toxic reactants, the simple reaction steps, the short reaction time and the high yield and purity of the product.

The invention is further illustrated by the following Examples.

### Example 1

### 6,7-Dichloro-1,5-dihydroimidazo[2,1-b]quinazolin-2[3H]-one hydrochloride semihydrate

To a solution of 150 ml of ethylene glycol and 15 ml (0.15 moles) of cc. hydrochloric acid 24.5 g (0.075 moles) of ethyl-(2-cyanoimino-5,6-dichloro-1,2,3,4-tetrahydroquinazolin-3-yl)-acetate are added in small portions, within about 10 minutes, at 115°C, and the mixture is kept at the same temperature for further 20 minutes. Then it is cooled to room temperature and made neutral by the dropwise addition of 10% by weight potassium carbonate solution. The separated crystals are filtered off. Thus 17.75 g (93.0%) of 6,7-di-chloro-1,5-dihydroimidazo[2,1-b]quinazolin-2[3H]-one base are obtained (m.p. > 300°C, HPLC content: 99.5%), which is dissolved in a hot mixture of 150 ml of methanol and 150 ml of cc. ethanolic hydrogen chloride (hydrogen chloride content: 0.75 moles/1000 ml). The mixture is then cooled, the separated product is filtered off and washed with a slight amount of ethanol.

Yield: 17.4 (82.8%).

M.p.: > 300°C, HPLC content: 99.9%.

### Example 2

### 6,7-Dichloro-1,5-dihydroimidazo[2,1-b]quinazolin-2[3H]-one hydrochloride semihydrate

To a solution of 10 ml of ethylene glycol in 0.5 ml (0.005 moles) of cc. hydrochloric acid, warmed to 120°C, 0.28 g (0.001 mole) of (2-cyanoimino-5,6-dichloro-1,2,3,4-tetrahydroquinazolin-3-yl)-acetonitrile are added, and the solution is stirred at the same temperature for 20 minutes. Then it is cooled to room temperature and made neutral with 10% potassium carbonate solution. The separated crystals are filtered off. Thus 0.27 g (96.4%) of crude 6,7-dichloro-1,5-dihydroimidazo[2,1-b]quinazolin-2[3H]-one base are obtained (m.p. > 300°C, HPLC content: 96.3%), which is dissolved in a hot mixture of 7 ml of methanol and 6 ml of cc. ethanolic hydrogen chloride (hydrogen chloride content: 0.76 moles/1000 ml) and allowed to crystallize. Then it is cooled, the separated product is filtered off and washed with a slight amount of ethanol.

Yield: 0.23 g (78.2%).

M.p.: > 300°C, HPLC content: 99.9%.

### Example 3

### 6,7-Dichioro-1,5-dihydroimidazo[2,1-b]quinazolin-2[3H]-one hydrochloride semihydrate

To a mixture of 15 ml of ethylene glycol and 2 ml (0.02 moles) of cc. hydrochloric acid, warmed to 120°C, 2.50 g (0.01 mole) of (2-cyanoimino-5,6-dichloro-1,2,3,4-tetrahydroquinazolin-3-yl)-acetonitrile are added, and the solution is stirred at the same temperature for 20 minutes. Then it is cooled to room temperature and made neutral with 10% potassium carbonate solution. The separated crystals are filtered off. Thus 2.72 g (97.1%) of crude 6,7-dichloro-1,5dihydroimidazo[2,1-b]quinazolin-2[3H]-one base are obtained (m.p. > 300°C, HPLC content: 97.2%), which is dissolved in a hot mixture of 15 ml of methanol and 16 ml of cc. ethanolic hydrogen chloride (hydrogen chloride content: 0.76 moles/1000 ml) and allowed to crystallize. Then it is cooled, the separated product is filtered off and washed with a slight amount of ethanol.

Yield: 2.56 (84.5%).

M.p.: > 300°C, HPLC content: 99.2%.

### Example 4

### 6,7-Dichloro-1,5-dihydroimidazo[2,1-b]quinazolin-2[3H]-one

0.163 g (0.0005 moles) of ethyl (2-cyanoimino-5,6-dichloro-1,2,3, 4-tetrahydroquinazolin-3-yl)-acetate are added to 2 ml of acetic acid at room temperature, the suspension thus obtained is heated to 100°C under stirring and kept at the same temperature for 30 minutes. Then it is cooled, the separated crystals are filtered off and washed with acetonitrile.

Yield: 0.116 g (91%).

M.p.: > 300°C, HPLC content: 99.15%.

### Example 5

### 6,7-Dichloro-1,5-dihydroimidazo[2,1-b]quinazolin-2[3H]-one hydrochloride semihydrate

0.2 ml of cc. hydrochloric acid and 0.163 g (0.0005 moles) of ethyl-(2-cyanoimino-5,6-dichloro-1,2,3,4-tetrahydroquinazolin-3-yl)-acetate are added to 2 ml of diethylene glycol dimethyl ether at room temperature. The reaction mixture is warmed to 110°C and kept at the same temperature for 60 minutes. The solution is then cooled, the separated crystals are filtered off and washed with ethanol.

Yield: 0.121 g (80%),

HPLC content: 86%.

The mother liquor is made alkaline (pH=8) with triethylamine, the free base thus obtained is filtered off and washed with ethanol.

Yield: 0.010 g (7.8%).

HPLC content: 90% .

Total yield: 87.8%.

### Example 6

### 6,7-Dichloro-1,5-dihydroimidazo[2,1-b]quinazolin-2[3H]-one hydrochloride semihydrate

One proceeds according to Example 5 except that diethylene glycol diethyl ether is used instead of diethylene glycol dimethyl ether.

Yield: 0.123 g (81%).

HPLC content: 87.5%.

The yield of the free base obtained from the mother liquor: 0.011 g (8.2%), HPLC content: 91.3%. Total yield: 89.2%.

### Example 7

### 6,7-Dichloro-1,5-dihydroimidazo[2,1-b]quinazolin-2[3H]-one hydrochloride semihydrate

One proceeds according to Example 5 except that instead of diethylene glycol dimethyl ether ethylene glycol dimethyl ether is used, and the reaction is carried out at 90°C for 100 minutes.

Yield: 0.124 g (81.8%).

HPLC content: 88.2%.

The yield of the free base obtained from the mother liquor: 0.014 g (11%), HPLC content: 93%. Total yield: 92.8%.

### Example 8

### 6,7-Dichloro-1,5-dihydroimidazo[2,1-b]quinazolin-2[3H]-one hydrochloride semihydrate

One proceeds according to Example 5 except that instead of diethylene glycol dimethyl ether 2-methoxyethanol is used, and the reaction mixture is allowed to crystallize at 0°C instead of room temperature.

Yield: 0.100 9 (66%)

HPLC content: 79%.

The yield of the free base obtained from the mother liquor: 0.01 g (7.8%). Total yield: 73.8%.

### Example 9

### 6,7-Dichloro-1,5-dihydroimidazo[2,1-b]quinazolin-2[3H]-one hydrochloride semihydrate

One proceeds according to Example 8 except that instead of 2-methoxyethanol 2-ethoxyethanol is used, and the reaction mixture is allowed to react for 75 minutes instead of 60 minutes.

Yield: 0.106 g (70%),

The mother liquor is made alkaline with cc. ammonia to obtain 0.013 g (10.3%) of free base. HPLC content: 87.3%. Total yield: 80.3%.

### Example 10

### 6,7-Dichloro-1,5-dihydroimidazo[2,1-b]quinazolin-2[3H]-one

0.163 g (0.0005 moles) of ethyl-(2-cyanoimino-5,6-dichloro-1,2,3,4-tetrahydroquinazolin-3-yl)-acetate and 0.2 ml of cc. hydrochloric acid are added to 0.2 ml of dimethylformamide, the suspension thus obtained is heated to 120°C under stirring and kept at the same temperature for 180 minutes. During the reaction further portions of hydrochloric acid are added to the mixture every 60 minutes, in a total amount of 0.2 ml. The solution thus obtained is cooled, rendered alkaline (pH=8) with triethyl amine and allowed to stand overnight in a refrigerator. The separated crystals are filtered off and washed with acetonitrile.

Yield: 0.070 g (54.7%), HPLC content: 87.3%.

### Example 11

### 6,7-Dichioro-1,5-dihydroimidazo[2,1-b]quinazolin-2[3H]-one

One proceeds according to Example 10 except that instead of dimethylformamide N-methylpyrrolidone is used, and the reaction is carried out at 130°C for 120 minutes.

Yield: 0.076 g (59.4%), HPLC content: 87.8%.

### Example 12

### 6,7-Dichloro-1,5-dihydroimidazo[2,1-b]quinazolin-2[3H]-one hydrochloride semihydrate

0.140 g (0.0005 moles) of (2-cyanoimino-5,6-dichloro-1,2,3,4-tetrahydroquinazolin-3-yl)-acetonitrile and 0.2 ml of cc. hydrochloric acid are added to 2 ml of acetonitrile. The reaction mixture is heated to 80°C under stirring and kept at the same temperature for 50 minutes. Then it is cooled, the separated crystals are filtered off and washed with acetonitrile.

Yield: 0.138 g (91.1%), HPLC content: 99.0%.

### Example 13

### 6,7-Dichloro-1,5-dihydroimidazo[2,1-b]quinazolin-2[3H]-one semihydrate

One proceeds according to Example 12 except that instead of acetonitrile dioxane is used, and the reaction mixture is stirred at 100°C for 30 minutes.

Yield: 0.139 g (91.7%), HPLC content: 99.2%.

### Example 14

### 6,7-Dichloro-1,5-dihydroimidazo[2,1-b]quinazolin-2[3H]-one

One proceeds according to Example 12 except that instead of acetonitrile dimethylformamide is used, and the reaction mixture is stirred at 100°C for 100 minutes. During the reaction further portions of cc. hydrochloric acid (2x0.2 ml) are added after 30 and 60 minutes, respectively. The mixture is then cooled, rendered alkaline with 10% by weight potassium carbonate solution (pH=8), the separated crystals are filtered off and washed with a slight amount of water and acetonitrile.

Yield: 0.080 g (62.5%), HPLC content: 88.%.

### Example 15

### 6,7-Dichloro-1,5-dihydroimidazo[2,1-b]quinazolin-2[3H]-one

One proceeds according to Example 14 except that instead of dimethylformamide N-methylpyrrolidone is used.

Yield: 0.078 g (61.0%), HPLC content: 87.3%.

### Example 16

### 6,7-Dichloro-1,5-dihydroimidazo[2,1-b]quinazolin-2[3H]-one hydrochloride semihydrate

0.140 g (0.0005 moles) of (2-cyanoimino-5,6-dichloro-1,2,3,4-tetrahydroquinazolin-3-yl)-acetonitrile and 0.2 ml of cc. hydrochloric acid are added to 2 ml of 2-methoxyethanol, and the mixture is reacted at 100°C for 50 minutes. Then it is cooled and allowed to stand overnight in a refrigerator. Then the separated crystals are filtered off and washed with acetonitrile.

Yield: 0.110 g (72.6%), HPLC content: 82%.

The filtrate is made alkaline (pH=8) with cc. ammonia solution, the separated crystals are filtered and washed with water and acetonitrile.

Yield of the free base obtained from the mother liquor: 0.011 g (8.6%), HPLC content: 87.2%.

Total yield: 81.2%.

### Example 17

### 6,7-Dichloro-1,5-dihydroimidazo[2,1-b]quinazolin-2[3H]-one hydrochloride semihydrate

One proceeds according to Example 16 except that instead of 2-methoxyethanol 2-ethoxyethanol is used.

Yield: 0.115 g (76.0%), HPLC content: 84.5%.

Yield of the free base obtained from the mother liquor: 0.011 g (8.6%), HPLC content: 88.3%.

Total yield: 84.6%.

### Example 18

### 6,7-Dichloro-1,5-dihydroimidazo[2,1-b]quinazolin-2[3H]-one hydrochloride semihydrate

One proceeds according to Example 16 except that instead of 2-methoxyethanol ethylene glycol dimethyl ether is used, and the reaction mixture is kept at 90°C for 70 minutes.

Yield: 0.128 g (84.5%), HPLC content: 92.5%.

Upon adding triethylamine to the mother liquor 0.011 g (8.6%) of 6,7-dichloro-1,5-dihydroimidazo[2,1-b]quinazolin-2[3H]-one base is obtained. HPLC content: 95.7%, total yield: 93.1%.

### Example 19

### 6,7-Dichloro-1,5-dihydroimidazo[2,1-b]quinazolin-2[3H]-one hydrochloride semihydrate

One proceeds as specified in Example 18 except that diethyleneglycol dimethyl ether is used instead of ethylene glycol dimethyl ether.

Yield: 0.127 g (83.8%), HPLC content: 92.3%.

Yield of the free base obtained from the mother liquor: 0.011 g (8.6%), HPLC content: 95.8%.

Total yield: 92.4%.

### Example 20

### 6,7-Dichloro-1,5-dihydroimidazo[2,1-b]quinazolin-2[3H]-one hydrochloride semihydrate

One proceeds as specified in Example 18 except that instead of 2-methoxyethanol diethylene glycol diethyl ether is used.

Yield: 0.125 g (82.5%), HPLC content: 92.0%.

Yield of the free base obtained from the mother liquor: 0.011 g (8.6%), HPLC content: 94.9%.

Total yield: 91.1%.

### Example 21

### 6,7-Dichloro-1,5-dihydroimidazo[2,1-b]quinazolin-2[3H]-one hydrochloride semihydrate

0.140 g (0.0005 moles) of (2-cyanoimino-5,6-dichloro-1,2,3,4-tetrahydroquinazolin-3-yl)-acetonitrile and 0.2 ml of water are added to 2 ml of acetic acid. The reaction mixture is heated to 80°C under stirring and kept at the same temperature for 30 minutes. Then it is cooled, the separated crystals are filtered off and washed with acetonitrile.

Yield: 0.118 g (92.2%),

HPLC content: 99.3%.

### Example 22

### Ethyl-(2-cyanoimino-5,6-dichloro-1,2,3,4-tetrahydroquinazolin-3-yl)-acetate

To a suspension of 2.38 g (0.01 mole) of diphenyl-N-cyanoimidocarbonate in 20 ml of acetonitrile a solution of 2.76 g (0.01 mole) of ethyl-(2-amino-5,6-dichlorobenzylamino)-acetate in 20 ml of acetonitrile are added dropwise at 20 to 30°C, within about 30 minutes under stirring. The reaction mixture is stirred further for 1 hour at room temperature, the separated product is filtered off and washed with a slight amount of acetonitrile.

Yield: 1.87 g (57.4%).

M.p.: 274-278°C (HPLC content: 99.2%).

The mother liquor is treated while hot with charcoal and evaporated to dryness in vacuo. The residue is dissolved in 10 ml of ethyl acetate, the solution is extracted first three times with 50 ml portions of 10% by weight potassium carbonate solution each, then twice with 5 ml of water each, dried, evaporated in vacuo and allowed to crystallize. The separated product is filtered off to yield further 0.66 g (20.2%) of the title compound.

M.p.: 275-278°C (HPLC content: 99.5%)

Total yield: 77.6%.

### Example 23

### Ethyl-(2-cyanoinino-5,6-dichloro-1,2,3,4-tetrahydroquinazolin-3-yl)-acetate

To a solution of 0.28 g (0.001 mole) of ethyl-(2-amino-5,6-dichlorobenzylamino)-acetate (GC content: 82%) in 2 ml of benzene 0.24 g (0.001 mole) of diphenyl-N-cyanoimidocarbonate is added at room temperature under stirring, and the reaction mixture is stirred at room temperature for 20 minutes. The separated product is filtered off and washed with a slight amount of benzene.

Yield: 0.21 g (64.4%).

M.p.: 263-270°C (HPLC content: 98.2%).

The product is recrystallized from 4 ml of dimethylformamide to yield 0.17 g (52%) of pure title compound.

M.p.: 268-275°C (HPLC content: 99.9%)

### Example 24

### Ethyl-(2-cyanoimino-5,6-dichloro-1,2,3,4-tetrahydroquinazolin-3-yl)-acetate

One proceeds as described in Example 23 except that instead of benzene 2 ml of dimethylformamide are used as solvent.

Yield: 0.19 g (58.3%).

M.p.: 271-278°C (HPLC content: 99.7%).

### Example 25

### Ethyl-(2-cyanoimino-5,6-dichloro-1,2,3,4-tetrahydroquinazolin-3-yl)-acetate

One proceeds as described in Example 23 except that instead of benzene 2 ml of isopropanol are used as solvent.

Yield: 0.23 g (70.6%).

M.p.: 268-274°C (HPLC content: 99.5%).

### Example 26

### Ethyl-(2-cyanoimino-5,6-dichloro-1,2,3,4-tetrahydroquinazolin-3-yl)-acetate

To a solution of 7.68 g (0.023 moles) of ethyl-(2-amino-5,6-dichlorobenzylamino)-acetate (GC content: 82%) in 50 ml of isopropanol 3.8 g (0.0026 moles) of dimethyl-N-cyanoimidocarbonate are added, and the reaction mixture is boiled for 3 hours under stirring. Then it is cooled, the separated product is filtered off and washed with a slight amount of cold isopropanol.

Yield: 2.8 g (37.3%).

M.p.: 272-278°C (HPLC content: 99.5%).

### Example 27

### (2-Cyanoimino-5,6-dichloro-1,2,3,4-tetrahydroquinazolin-3-yl)-acetonitrile

To a suspension of 5.0 g (0.0208 moles) of diphenyl-N-cyanoimidocarbonate in 100 ml of acetonitrile 4.61 g (0.02 moles) of (2-amino-5,6-dichlorobenzylamino)-acetonitrile are added, the reaction mixture is heated to 60°C within 20 minutes under stirring and allowed to react at the same temperature for further 1 hour. Then it is cooled, the separated product is filtered off and washed with a slight amount of acetonitrile.

Yield: 3.53 g (60.9%).

M.p.: >280°C (HPLC content: 99.5%).

The filtrate is evaporated to about a third of its original volume to yield 1.2 g (20.7%) of the title compound.

M.p.: >280°C (HPLC content: 98.7%).

Total yield: 81.6%.

### Example 28

### Benzyl-(2-cyanoimino-5,6-dichloro-1,2,3,4-tetrahydroquinazolin-3-yl)-acetate

To a suspension of 7.2 g (0.030 moles) of diphenyl-N--cyanoimidocarbonate in 120 ml of acetonitrile 10.20 g (0.030 moles) of benzyl-(2-amino-5,6-dichlorobenzylamino)-acetate are added, the reaction mixture is heated to 60°C within 20 minutes under stirring and allowed to react at the same temperature for further 1 hour. Then it is cooled, the separated product is filtered off and washed with a slight amount of acetonitrile and acetone.

Yield: 6.94 g (60.0%).

M.p.: 278-285°C (HPLC content: 98.9%).

The filtrate is evaporated to about a third of its original volume to yield further 2.0 g (17.3%) of product.

M.p.: 279-284°C (HPLC content: 98.4%).

Total yield: 77.3%.

### Example 29

### Ethyl-(2-cyanoimino-5,6-dichloro-1,2,3,4-tetrahydroquinazolin-3-yl)-acetate

To a solution of 19.8 g (0.1 M) of dibenzyl-(N-cyanoimidodithiocarbonate) in 100 ml of dimethylformamide 27.7 g (0.1 M) of 6-amino-2,3-dichlorobenzylglycine ethyl ester are added, and the reaction mixture is stirred at 100°C for 5 hours. Then it is cooled, 100 ml of water are added thereto, and it is allowed to crystallize. The separated product is filtered off and washed with a slight amount of isopropanol.

Yield: 10.1 g (31%).

M.p.: 274-277°C.

## Claims (Claims for the following Contracting State(s): DE, FR, IT, NL, SE, LI, CH, BE, AT, DK)

1. A process for the preparation of 6,7-di-(chloro)-1,5-di-(hydro)-imidazo[2,1-b]quinazolin-2[3H]-one of formula 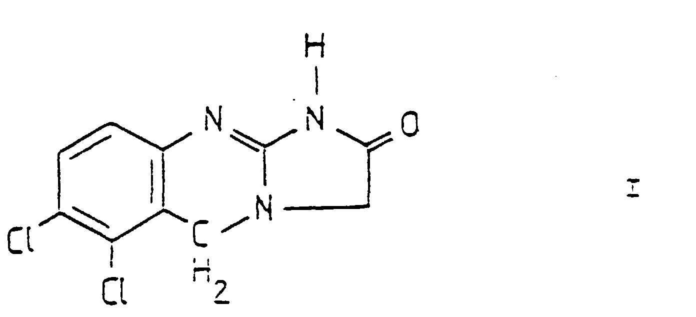 or a salt thereof including their hydrates, characterized, by subjecting [2-(cyanoimino)-5,6-di-(chloro)-1,2,3,4-tetra-(hydro)-quinazolin-3-yl]-acetonitrile or a [2-(cyanoimino)-5,6-di-(chloro)-1,2,3,4-tetra-(hydro)-quinazolin-3-yl]-acetic acid alkylester of the general formula 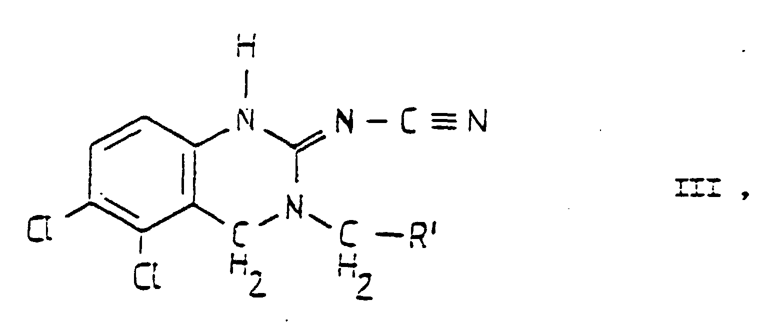 wherein
R' stands for a cyano group or a group of the formula 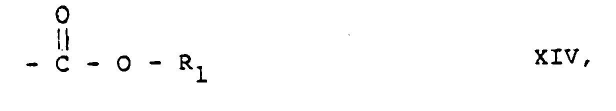 in which latter
R₁ represents a straight or branched alkyl group having from 1 to 6 carbon atom(s), optionally carrying a phenyl substituent,
to thermic cyclization in an acidic medium with ethylene glycol, ethylene glycol dimethyl ether, ethylene glycol diethyl ether, diethylene glycol dimethyl ether, diethylene glycol diethyl ether, 2-(methoxy)-ethanol, 2-(ethoxy)-ethanol, N-(methyl)-pyrrolidone, dimethylformamide, acetonitrile and/or dioxane and/or in an aliphatic organic acid having from 1 to 4 carbon atom(s) as [a] solvent(s).

2. A process as claimed in claim 1, characterized by carrying out the cyclization in a mixture of 1 or more solvent(s) inert towards the reactants and a mineral acid.

3. A process as claimed in any of claim 1 or 2, characterized by using as a mineral acid hydrogen chloride.

4. A process as claimed in any of claims 1 to 3, characterized by carrying out the reaction in an aliphatic organic acid having from 1 to 4 carbon atom(s) in acetic acid.

5. A process as claimed in any of claims 1 to 4, characterized by carrying out the reaction at a temperature from 80°C to 130°C.

6. [2-(Cyanoimino)-5,6-di-(chloro)-1,2,3,4-tetra-(hydro)-quinazolin-3-yl]-acetonitriles or -acetic acid alkylesters of the general formula 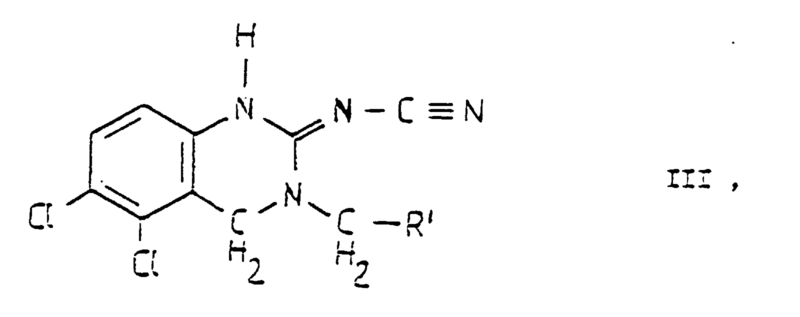 wherein
R' stands for a cyano group or a group of the formula 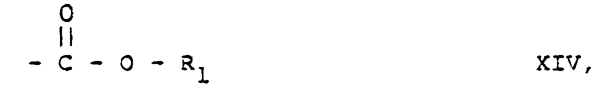 in which latter
R₁ represents a straight or branched alkyl group having from 1 to 6 carbon atom(s), optionally carrying a phenyl substituent.

7. A process for the preparation of the [2-(cyanoimino)-5,6-di-(chloro)-1,2,3,4-tetra-(hydro)-quinazolin-3-yl]-acetonitriles or -acetic acid alkylesters according to claim 6, characterized by reacting a [2-(amino)-5,6-di-(chloro)-benzylamino]-acetonitrile or a [2-(amino)-5,6-di-(chloro)-benzylamino]-acetic acid alkylester of the general formula 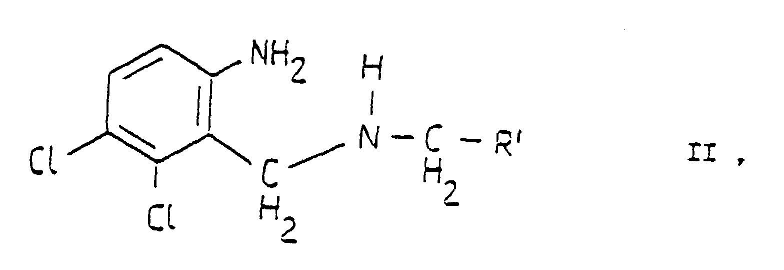 wherein
R' is as defined in claim 6,
with a cyanoimino derivative of the general formula 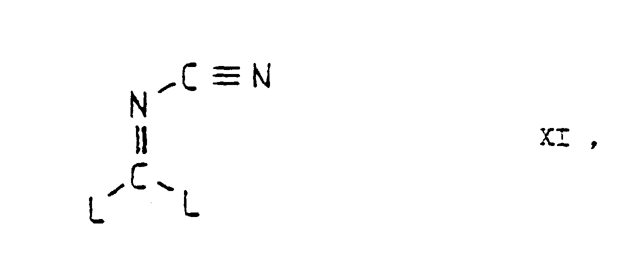 wherein
L is a leaving group.

8. A process as claimed in claim 7, characterized by using as cyanoimino derivative of the general formula XI such one,
in which
L stands for a group of formula
X - R₂ XV,
in which latter
X represents oxygen or sulfur and
R₂ means an alkyl group having from 1 to 6 carbon atom(s), optionally carrying a phenyl substituent.

9. A process as claimed in claim 7 or 8, characterized by carrying out the reaction in [a] solvent(s) inert towards the reactants, particularly in an alcohol having from 1 to 8 carbon atom(s), and/or [an] apolar and/or polar aprotic solvent(s).

10. A process as claimed in any of claims 7 to 9, characterized by using as solvent(s) isopropanol, benzene, acetonitrile and/or dimethylformamide.

11. A process as claimed in any of claims 7 to 10, characterized by carrying out the reaction at a temperature from 0°C to 100°C.

## Claims (Claims for the following Contracting State(s): ES, GR)

1. A process for the preparation of 6,7-di-(chloro)-1,5-di-(hydro)-imidazo[2,1-b]quinazolin-2[3H]-one of formula 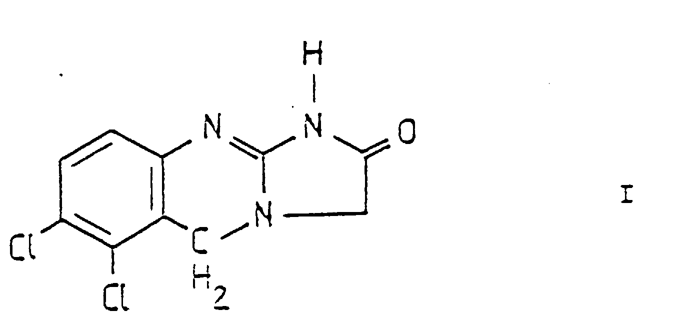 or a salt thereof including their hydrates, characterized by subjecting [2-(cyanoimino)-5,6-di-(chloro)-1,2,3,4-tetra-(hydro)-quinazolin-3-yl]-acetonitrile or a [2-(cyanoimino)-5,6-di-(chloro)-1,2,3,4-tetra-(hydro)-quinazolin-3-yl]-acetic acid alkylester of the general formula 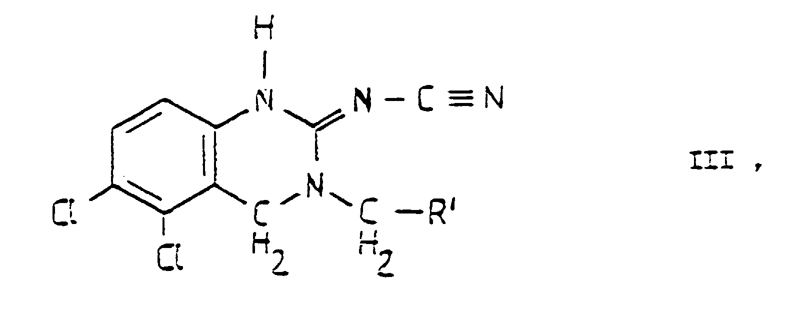 wherein
R' stands for a cyano group or a group of the formula 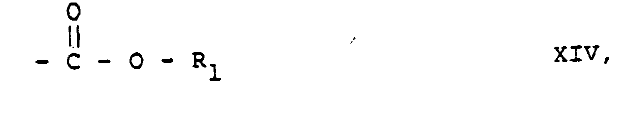 in which latter
R₁ represents a straight or branched alkyl group having from 1 to 6 carbon atom(s), optionally carrying a phenyl substituent,
to thermic cyclization in an acidic medium with ethylene glycol, ethylene glycol dimethyl ether, ethylene glycol diethyl ether, diethylene glycol dimethyl ether, diethylene glycol diethyl ether, 2-(methoxy-)-ethanol, 2-(ethoxy)-ethanol, N-(methyl)-pyrrolidone, dimethylformamide, acetonitrile and/ /or dioxane and/or in an aliphatic organic acid having from 1 to 4 carbon atom(s) as [a] solvent(s).

2. A process as claimed in claim 1, characterized by carrying out the cyclization in a mixture of 1 or more solvent(s) inert towards the reactants and a mineral acid.

3. A process as claimed in any of claim 1 or 2, characterized by using as a mineral acid hydrogen chloride.

4. A process as claimed in any of claims 1 to 3, characterized by carrying out the reaction in an aliphatic organic acid having from 1 to 4 carbon atom(s) in acetic acid.

5. A process as claimed in any of claims 1 to 4, characterized by carrying out the reaction at a temperature from 80°C to 130°C.

6. A process for the preparation of the [2-(cyanoimino)-5,6-di-(chloro)-1,2,3,4-tetra-(hydro)-quinazolin-3-yl]-acetonitriles or -acetic acid alkylesters of the general formula 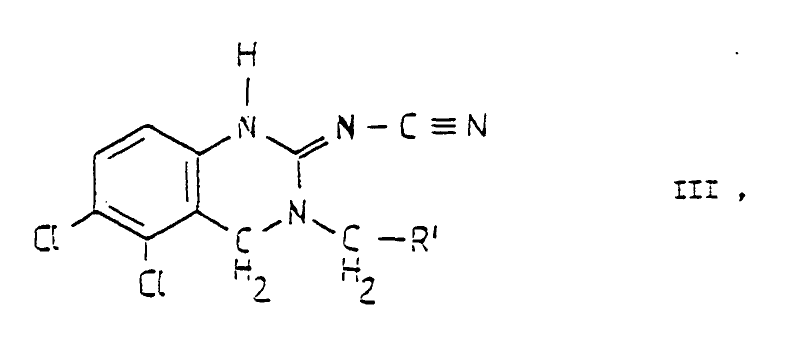 wherein
R' stands for a cyano group or a group of the formula 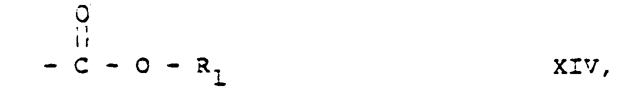 in which latter
R₁ represents a straight or branched alkyl group having from 1 to 6 carbon atom(s), optionally carrying a phenyl substituent,
characterized by reacting a [2-(amino)-5,6-di-(chloro)-benzylamino]-acetonitrile or a [2-(amino)-5,6-di-(chloro)-benzylamino]-acetic acid alkylester of the general formula 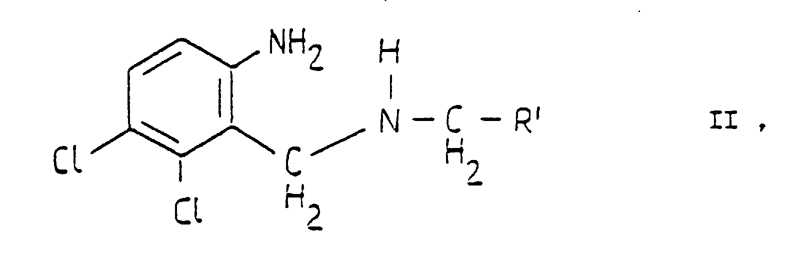 wherein
R' is as above defined
with a cyanoimino derivative of the general formula 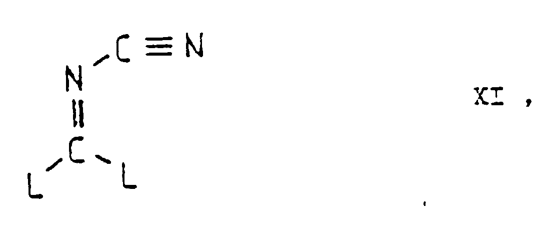 wherein
L is a leaving group.

7. A process as claimed in claim 6, characterized by using as cyanoimino derivative of the general formula XI such one,
in which
L stands for a group of formula
X - R₂ XV,
in which latter
X represents oxygen or sulfur and
R₂ means an alkyl group having from 1 to 6 carbon atom(s), optionally carrying a phenyl substituent.

8. A process as claimed in claim 6 or 7, characterized by carrying out the reaction in [a] solvent(s) inert towards the reactants, particularly in an alcohol having from 1 to 8 carbon atom(s), apolar and/or polar aprotic solvent(s).

9. A process as claimed in any of claims 6 to 8, characterized by using as solvent(s) isopropanol, benzene, acetonitrile and/or dimethylformamide.

10. A process as claimed in any of claims 6 to 9, characterized by carrying out the reaction at a temperature from 0°C to 100°C.

## Patentansprüche (Patentansprüche für folgende(n) Vertragsstaat(en): DE, FR, IT, NL, SE, LI, CH, BE, AT, DK)

1. Verfahren zur Herstellung von 6,7-Di-(chlor)-1,5 di-(hydro)-imidazo-[2,1-b]-chinazolin-2[3H]-on der Formel 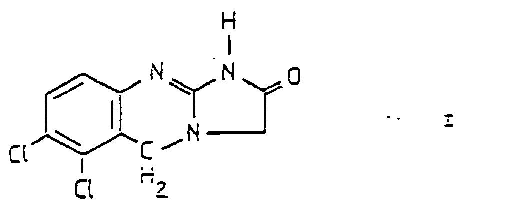 oder eines Salzes davon, einschließlich der Hydrate, **dadurch gekennzeichnet,** daß [2-(Cyanoimino)-5,6-di-(chlor)-1,2,3,4-tetra-(hydro)-chinazolin-2-yl]-acetonitril oder ein [2-(Cyanoimino)-5,6-di-(chlor)-1,2,3,4-tetra-(hydro)-chinazolin-3-yl]-essigsäurealkylester der allgemeinen Formel 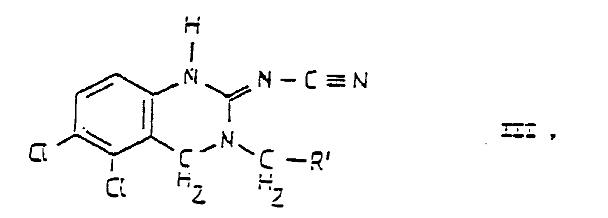 worin
R' für eine Cyanogruppe oder eine Gruppe der Formel 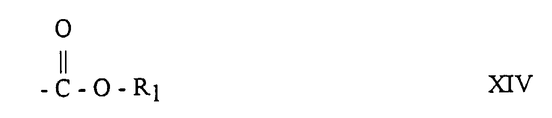 steht, wobei in letzterer
R₁ für eine geradkettige oder verzweigte Alkylgruppe mit 1 bis 6 Kohlenstoffatomen, wahlweise einen Phenylsubstituenten tragend, steht,
zur thermischen Cyclisierung in einem sauren Medium mit Ethylenglykol, Ethylenglykoldimethylether, Ethylenglykoldiethylether, Diethylenglykoldimethylether, Diethylenglykoldiethylether, 2-(Methoxy)-ethanol, 2-(Ethoxy)-ethanol, N-(Methyl)pyrrolidon, Dimethylformamid, Acetonitril und/oder Dioxan und/oder in einer aliphatischen organischen Säure mit 1 bis 4 Kohlenstoffatomen als (ein) Lösemittel umgesetzt wird.

2. Verfahren nach Anspruch 1, **dadurch gekennzeichnet,** daß die Cyclisierung in einer Mischung aus einem oder mehreren Lösemitteln, die gegenüber den Reaktanten inert sind, und einer Mineralsäure durchgeführt wird.

3. Verfahren nach einem der Ansprüche 1 oder 2, **dadurch gekennzeichnet,** daß als Mineralsäure Chlorwasserstoff verwendet wird.

4. Verfahren nach einem der Ansprüche 1 bis 3, **dadurch gekennzeichnet,** daß die Reaktion in einer aliphatischen organischen Säure mit 1 bis 4 Kohlenstoffatomen in Essigsäure durchgeführt wird.

5. Verfahren nach einem der Ansprüche 1 bis 4, **dadurch gekennzeichnet,** daß die Reaktion bei einer Temperatur von 80 bis 130°C durchgeführt wird.

6. [2-(Cyanoirnino)-5,6-di-(chlor)-1,2,3,4-tetra-(hydro)-chinazolin-3-yl]-acetonitrile oder -essigsäurealkylester der allgemeinen Formel 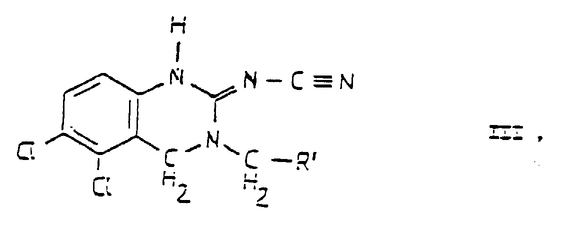 worin
R' für eine Cyanogruppe oder eine Gruppe der Formel 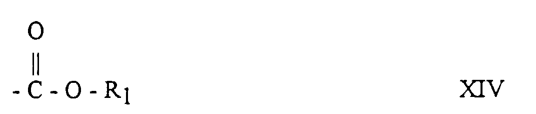 steht, wobei in letzterer
R₁ für eine geradkettige oder verzweigte Alkylgruppe mit 1 bis 6 Kohlenstoffatomen, wahlweise einen Phenylsubstituenten tragend, steht.

7. Verfahren zur Herstellung der Verbindung [2-(Cyanoimino)-5,6-di-(chlor)-1,2,3,4-tetra(hydro)-chinazolin-3-yl]-acetonitrile oder -essigsäurealkylester gemäß Anspruch 6, **dadurch gekennzeichnet,** daß ein [2-(Amino)-5,6-di-(chlor)-benzylamino]-acetonitril oder ein [2-(Amino)-5,6-di-(chlor)-benzylamino]-essigsäurealkylester der allgemeinen Formel 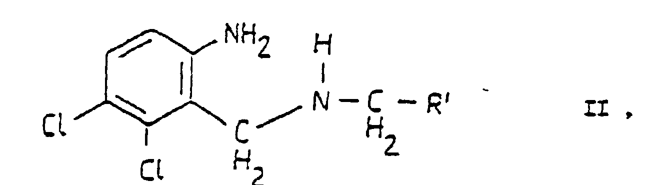 worin
R' die gleiche Bedeutung wie in Anspruch 6 hat,
mit einem Cyanoimino-Derivat der allgemeinen Formel 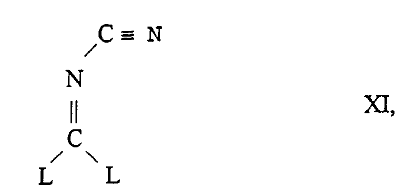 worin
L eine Abgangsgruppe ist,
umgesetzt wird.

8. Verfahren nach Anspruch 7, **dadurch gekennzeichnet,** daß als Cyanoimino-Derivat der allgemeinen Formel XI eines verwendet wird,
in dem
L für eine Gruppe der Formel
X - R₂ XV
steht, worin in dieser
X für Sauerstoff oder Schwefel steht
und
R₂ eine Alkylgruppe mit 1 bis 6 Kohlenstoffatomen, gegebenenfalls einen Phenylsubstituenten tragend, bedeutet.

9. Verfahren nach Anspruch 7 oder 8, **dadurch gekennzeichnet,** daß die Reaktion in (einem) Lösemittel(n) durchgeführt wird, welche gegenüber den Reaktanten inert sind, insbesondere in einem Alkohol mit 1 bis 8 Kohlenstoffatomen und/oder (einem) apolaren und/oder polaren aprotischen Lösemittel(n).

10. Verfahren nach einem der Ansprüche 7 bis 9, **dadurch gekennzeichnet,** daß als Lösemittel Isopropanol, Benzol, Acetonitril und/oder Dimethylformamid verwendet wird/werden.

11. Verfahren nach einem der Ansprüche 7 bis 10, dadurch gekennzeichnet, daß die Reaktion bei einer Temperatur von 0 bis 100°C durchgeführt wird.

## Patentansprüche (Patentansprüche für folgende(n) Vertragsstaat(en): ES, GR)

1. Verfahren zur Herstellung von 6,7-Di-(chlor)-1,5-di-(hydro)-imidazo-[2,1-b]-chinazolin-2[3H]-on der Formel 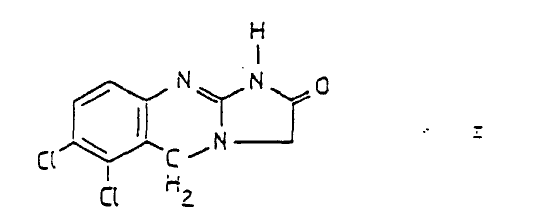 oder eines Salzes davon, einschließlich der Hydrate, **dadurch gekennzeichnet,** daß [2-(Cyanoimino)-5,6-di-(chlor)-1,2,3,4-tetra-(hydro)-chinazolin-2-yl]-acetonitril oder ein [2-(Cyanoimino)-5,6-di-(chlor)-1,2,3,4-tetra-(hydro)-chinazolin-3-yl]-essigsäurealkylester der allgemeinen Formel 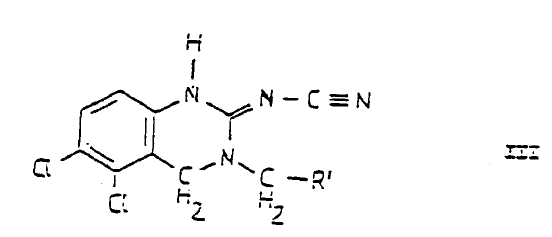 worin
R' für eine Cyanogruppe oder eine Gruppe der Formel 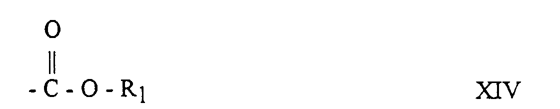 steht, wobei in letzterer
R₁ für eine geradkettige oder verzweigte Alkylgruppe mit 1 bis 6 Kohlenstoffatomen, wahlweise einen Phenylsubstituenten tragend, steht,
zur thermischen Cyclisierung in einem sauren Medium mit Ethylenglykol, Ethylenglykoldimethylether, Ethylenglykoldiethylether, Diethylenglykoldimethylether, Diethylenglykoldiethylether, 2-(Methoxy)-ethanol, 2-(Ethoxy)-ethanol, N-(Methyl)-pyrrolidon, Dimethylformamid, Acetonitril und/oder Dioxan und/oder in einer aliphatischen organischen Säure mit 1 bis 4 Kohlenstoffatomen als (ein) Lösemittel umgesetzt wird.

2. Verfahren nach Anspruch 1, **dadurch gekennzeichnet,** daß die Cyclisierung in einer Mischung aus einem oder mehreren Lösemitteln, die gegenüber den Reaktanten inert sind, und einer Mineralsäure durchgeführt wird.

3. Verfahren nach einem der Ansprüche 1 oder 2, **dadurch gekennzeichnet,** daß als Mineralsäure Chlorwasserstoff verwendet wird.

4. Verfahren nach einem der Ansprüche 1 bis 3, **dadurch gekennzeichnet,** daß die Reaktion in einer aliphatischen organischen Säure mit 1 bis 4 Kohlenstoffatomen in Essigsäure durchgeführt wird.

5. Verfahren nach einem der Ansprüche 1 bis 4, **dadurch gekennzeichnet,** daß die Reaktion bei einer Temperatur von 80 bis 130°C durchgeführt wird.

6. Verfahren zur Herstellung von [2-(Cyanoimino)-5,6-di-(chlor)-1,2,3,4-tetra-(hydro)-chinazolin-3-yl]-acetonitrilen oder -essigsäurealkylestern der allgemeinen Formel 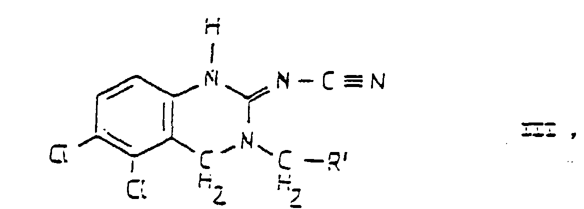 worin
R' für eine Cyanogruppe oder eine Gruppe der Formel 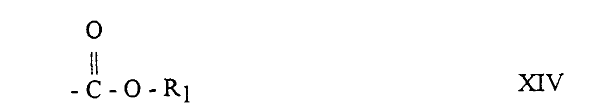 steht, wobei in letzterer
R₁ für eine geradkettige oder verzweigte Alkylgruppe mit 1 bis 6 Kohlenstoffatomen, wahlweise einen Phenylsubstituenten tragend, steht,
**dadurch gekennzeichnet,** daß ein [2-(Amino)-5,6-di-(chlor)-benzylamino]-acetonitril oder ein [2-(Amino)-5,6-di-(chlor)-benzylamino]-essigsäurealkylester der allgemeinen Formel 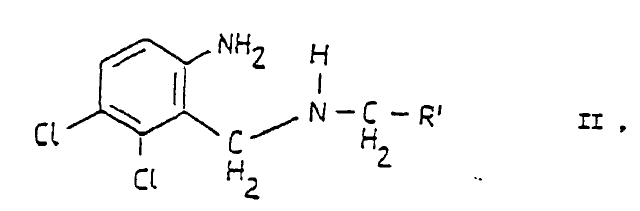 worin
R' die gleiche Bedeutung wie oben hat,
mit einem Cyanoimino-Derivat der allgemeinen Formel 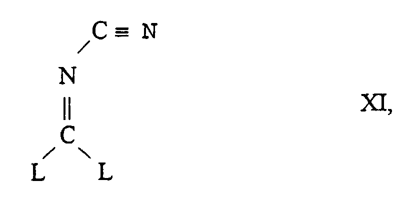 worin
L eine Abgangsgruppe ist,
umgesetzt wird.

7. Verfahren nach Anspruch 6, **dadurch gekennzeichnet,** daß als Cyanoimino-Derivat der allgemeinen Formel XI eines verwendet wird,
in dem
L für eine Gruppe der Formel
X - R₂ XV
steht, worin in dieser
X für Sauerstoffoder Schwefel steht
und
R₂ eine Alkylgruppe mit 1 bis 6 Kohlenstoffatomen, gegebenenfalls einen Phenylsubstituenten tragend, bedeutet.

8. Verfahren nach Anspruch 6 oder 7, **dadurch gekennzeichnet,** daß die Reaktion in (einem) Lösemittel(n) durchgeführt wird, welche gegenüber den Reaktanten inert sind, insbesondere in einem Alkohol mit 1 bis 8 Kohlenstoffatomen und/oder (einem) apolaren und/oder polaren aprotischen Lösemittel(n).

9. Verfahren nach einem der Ansprüche 6 bis 8, **dadurch gekennzeichnet,** daß als Lösemittel Isopropanol, Benzol, Acetonitril und/oder Dimethylformamid verwendet wird/werden.

10. Verfahren nach einem der Ansprüche 6 bis 9, dadurch gekennzeichnet, daß die Reaktion bei einer Temperatur von 0 bis 100°C durchgeführt wird.

## Revendications (Revendications pour l'(les) Etat(s) contractant(s) suivant(s): DE, FR, IT, NL, SE, LI, CH, BE, AT, DK)

1. Un procédé de préparation de la 6,7-di-(chloro)-1,5-di-(hydro)-imidazo-[2,1-b]-quinazoline-2[3H]-one de formule: 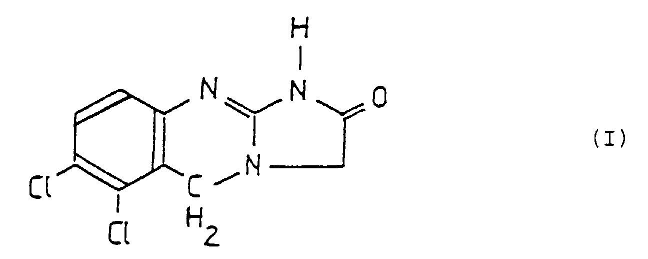 ou un de ses sels y compris leurs hydrates, caractérisé en ce que l'on soumet la [2-(cyanoimino)-5,6-di-(chloro)-1,2,3,4-tétra-(hydro)-quinazoline-3-yl] acétonitrile ou une [2-(cyanoimino)-5,6-di-(chloro)-1,2,3,4-tétra-(hydro)-quinazoline-3-yl] acétate d'alkyle de formule générale: 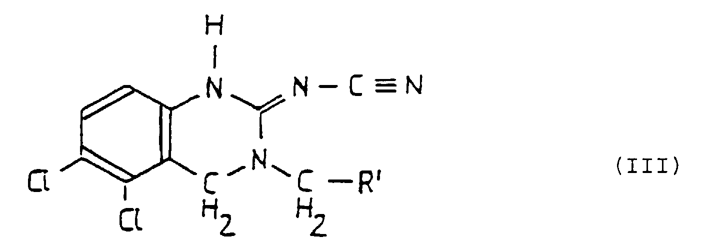 dans laquelle:
R' représente un groupe cyano ou un groupe de formule: 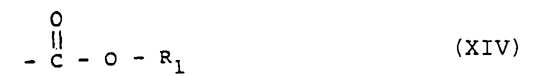 dans laquelle:
R₁ représente un groupe alkyle linéaire ou ramifiée comportant de 1 à 6 atomes de carbone, portant éventuellement un substituant phényle,
à une cyclisation thermique en milieu acide en présence d'éthylène glycol, d'éthylène glycol diméthyl éther, d'éthylène glycol diéthyl éther, diéthylène glycol diméthyl éther, diéthylène glycol diéthyl éther, 2-(méthoxy) -éthanol, 2- (éthoxy) -éthanol, N- (méthyl) -pyrrolidone, diméthylformamide, acétonitrile et/ou dioxane et/ou dans un acide organique aliphatique comportant de 1 à 4 atomes de carbone en tant que solvants.

2. Un procédé selon la revendication 1, caractérisé en ce que l'on effectue la cyclisation dans un mélange de un ou plusieurs solvants inertes vis-à-vis des réactifs et d'un acide minéral.

3. Un procédé selon une quelconque des revendications 1 ou 2, caractérisé en ce que l'on utilise en tant qu'acide minéral de l'acide chlorhydrique.

4. Un procédé selon une quelconque des revendications 1 à 3, caractérisé en ce que l'on effectue la réaction dans un acide organique aliphatique comportant 1 à 4 atomes de carbone dans l'acide acétique.

5. Un procédé selon une quelconque des revendications 1 à 4, caractérisé en ce que l'on effectue la réaction à une température comprise entre 80 et 130°C.

6. 2-(cyanoimino)-5,6-di-(chloro)-1,2,3,4-tétra-(hydro)-quinazoline-3-yl)-acétonitrile ou acétate d'alkyle de formule générale: 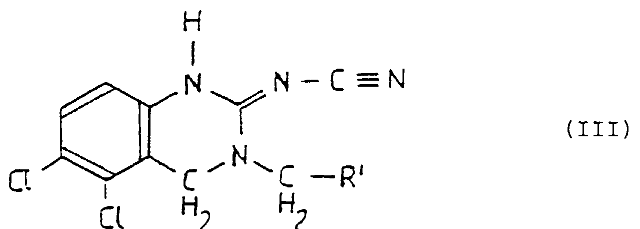 dans laquelle:
R' représente un groupe cyano ou un groupe de formule: 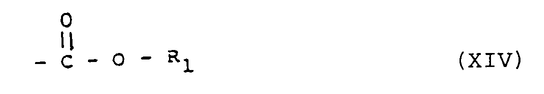 où:
R₁ représente un groupe alkyle linéaire ou ramifié comportant 1 à 6 atomes de carbone, comportant éventuellement un phényle comme substituant.

7. Un procédé de préparation de [2-(cyanoimino)-5,6-di-(chloro)-1,2,3,4-tétra-(hydro)-quinazoline-3-yl]-acétonitriles ou acétate d'alkyle selon la revendication 6, caractérisé en ce que l'on fait réagir une [2-(amino)-5,6-di-(chloro)-benzylamino]-acétonitrile ou un [2-(amino) -5,6-di- (chloro) -benzylamino] -acétate d'alkyle de formule générale: 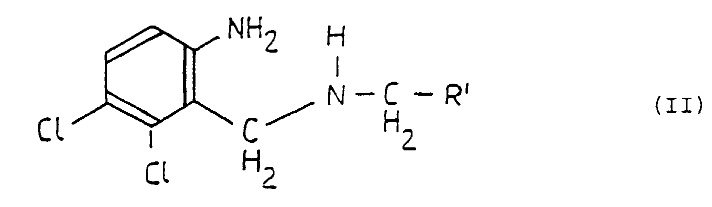 où:
R' est tel que défini dans la revendication 6, avec un dérivé cyanoimino de formule générale: 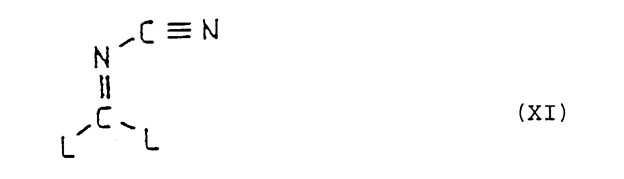 dans laquelle:
L est un groupe partant.

8. Un procédé selon la revendication 7, caractérisé en ce que l'on utilise en tant que dérivé cyanoimino de formule générale XI, un composé tel que:
L représente un groupe de formule:
X - R₂ (XV)
dans laquelle:
X représente l'oxygène ou le soufre, et R₂ représente un groupe alkyle comportant de 1 à 6 atomes de carbone, portant éventuellement un substituant phényle.

9. Un procédé selon la revendication 7 ou 8, caractérisé en ce que l'on effectue la réaction dans un ou plusieurs solvants inertes vis-à-vis des réactifs, en particulier dans un alcool comportant de 1 à 8 atomes de carbone et/ou un ou plusieurs solvants aprotiques apolaires et/ou polaires.

10. Un procédé selon l'une quelconque des revendications 7 à 9 caractérisé en ce que l'on utilise en tant que solvant, l'isopropanol, le benzène, l'acétonitrile et/ou le diméthylformamide.

11. Un procédé selon l'une quelconque des revendications 7 à 10, caractérisé en ce que l'on effectue la réaction à une température de 0 à 100°C.

## Revendications (Revendications pour l'(les) Etat(s) contractant(s) suivant(s): ES, GR)

1. Un procédé de préparation de la 6,7-di-(chloro)-1,5-di-(hydro)-imidazo-[2,1-b]-quinazoline-2[3H]-one de formule: 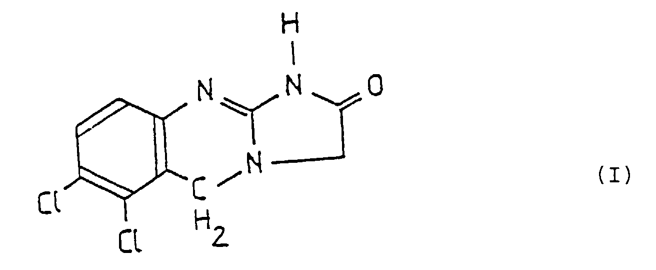 ou un de ses sels y compris leurs hydrates, caractérisé en ce que l'on soumet la [2-(cyanoimino)-5,6-di-(chloro)-1,2,3,4-tétra-(hydro)-quinazoline-3-yl] acétonitrile ou une [2-(cyanoimino)-5,6-di-(chloro)-1,2,3,4-tétra-(hydro)-quinazoline-3-yl] acétate d'alkyle de formule générale: 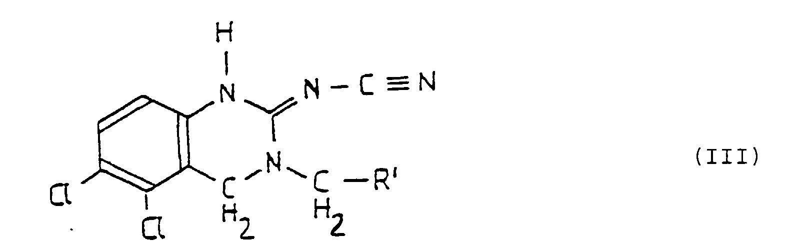 dans laquelle:
R' représente un groupe cyano ou un groupe de formule: 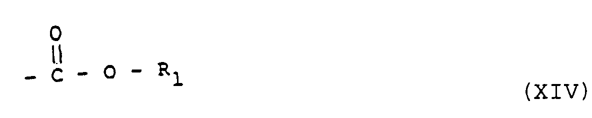 dans laquelle:
R₁ représente un groupe alkyle linéaire ou ramifiée comportant de 1 à 6 atomes de carbone, portant éventuellement un substituant phényle,
à une cyclisation thermique en milieu acide en présence d'éthylène glycol, d'éthylène glycol diméthyl éther, d'éthylène glycol diéthyl éther, diéthylène glycol diméthyl éther, diéthylène glycol diéthyl éther, 2-(méthoxy)-éthanol, 2-(éthoxy)-éthanol, N-(méthyl)-pyrrolidone, diméthylformamide, acétonitrile et/ou dioxane et/ou dans un acide organique aliphatique comportant de 1 à 4 atomes de carbone en tant que solvants.

2. Un procédé selon la revendication 1, caractérisé en ce que l'on effectue la cyclisation dans un mélange de un ou plusieurs solvants inertes vis-à-vis des réactifs et d'un acide minéral.

3. Un procédé selon une quelconque des revendications 1 ou 2, caractérisé en ce que l'on utilise en tant qu'acide minéral de l'acide chlorhydrique.

4. Un procédé selon une quelconque des revendications 1 à 3, caractérisé en ce que l'on effectue la réaction dans un acide organique aliphatique comportant 1 à 4 atomes de carbone dans l'acide acétique.

5. Un procédé selon une quelconque des revendications 1 à 4, caractérisé en ce que l'on effectue la réaction à une température comprise entre 80 et 130°C.

6. Un procédé de préparation de 2-(cyanoimino)-5,6-di-(chloro)-1,2,3,4-tétra-(hydro)-quinazoline-3-yl)-acétonitrile ou acétate d'alkyle de formule générale: 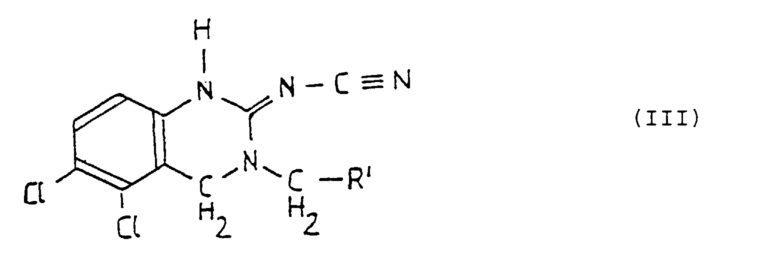 dans laquelle:
R' représente un groupe cyano ou un groupe de formule: 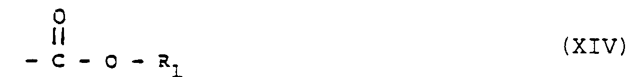 où:
R₁ représente un groupe alkyle linéaire ou ramifié comportant 1 à 6 atomes de carbone, portant éventuellement un phényle comme substituant,
caractérisé en ce que l'on fait réagir une [2-(amino)-5,6-di-(chloro)-benzylamino]-acétonitrile ou un [2-(amino)-5,6-di-(chloro)-benzylamino]-acétate d'alkyle de formule générale: 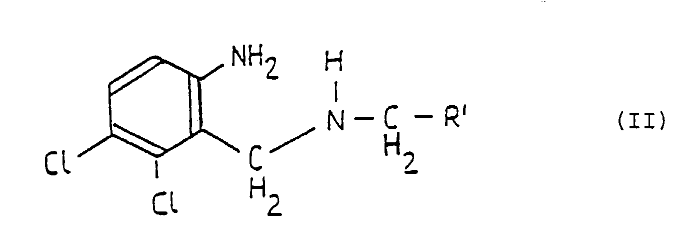 où:
R' est tel que défini ci-dessus, avec un dérivé cyanoimino de formule générale: 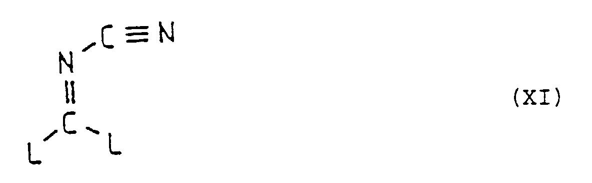 dans laquelle:
L est un groupe partant.

7. Un procédé selon la revendication 6, caractérisé en ce que l'on utilise en tant que dérivé cyanoimino de formule générale XI, un composé dans lequel:
L représente un groupe de formule:
X - R₂ (XV)
et dans laquelle:
X représente l'oxygène ou le soufre, et R₂ représente un groupe alkyle comportant de 1 à 6 atomes de carbone, portant éventuellement un substituant phényle.

8. Un procédé selon la revendication 6 ou 7, caractérisé en ce que l'on effectue la réaction dans un ou plusieurs solvants inertes vis-à-vis des réactifs, en particulier dans un alcool comportant de 1 à 8 atomes de carbone et/ou un ou plusieurs solvants aprotiques apolaires et/ou polaires.

9. Un procédé selon l'une quelconque des revendications 6 à 8 caractérisé en ce que l'on utilise en tant que solvant, l'isopropanol, le benzène, l'acétonitrile et/ou le diméthylformamide.

10. Un procédé selon l'une quelconque des revendications 6 à 9, caractérisé en ce que l'on effectue la réaction à une température de 0 à 100°C.
